(19) Europäisches Patentamt

European Patent Office

Office européen des brevets

(11) **EP 1 025 876 A2**

(12) **EUROPÄISCHE PATENTANMELDUNG**

(43) Veröffentlichungstag:
**09.08.2000  Patentblatt 2000/32**

(51) Int. Cl.[7]: **A61M 25/06**, A61M 25/00

(21) Anmeldenummer: **00101068.5**

(22) Anmeldetag: **20.01.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **06.02.1999 DE 19904896**

(71) Anmelder: **Convergenza AG**
**9490 Vaduz (LI)**

(72) Erfinder:
- **Nellessen, Thomas**
  **78464 Konstanz (DE)**
- **Stevens, Klaus**
  **52152 Simmerath (DE)**

(74) Vertreter:
**Vetter, Ewald Otto et al**
**Meissner, Bolte & Partner**
**Anwaltssozietät GbR**
**Postfach 10 26 05**
**86016 Augsburg (DE)**

(54) **Kanüle**

(57)      Kanüle zur Blutzufuhr in eine Blutbahn, insbesondere die Aorta, eines Patienten. Die Kanüle hat an ihrem distalen Ende (6) eine Vielzahl von Blutauslaßöffnungen (14). Der Strömungsquerschnitt der einzelnen Blutauslaßöffnungen ist je maximal 0,09 mm$^2$ groß. Die Summe der Strömungsquerschnitte von allen Blutauslaßöffnungen (14) zusammen ist mindestens so groß wie der Strömungsquerschnitt der Kanüle an ihrem distalen Ende.

Fig.1

**EP 1 025 876 A2**

**Beschreibung**

Die Erfindung betrifft eine Kanüle gemäß dem Oberbegriff von Anspruch 1 zur Blutzufuhr in eine Blutbahn eines Patienten, insbesondere in die Aorta eines Patienten während einer Herzoperation.

Eine Kanüle für Blutzufuhr in die Aorta während einer Herzoperation ist aus dem US-Patent 5 685 865 bekannt. In ihr ist beschrieben, daß Aortenkanülen dazu benutzt werden, Blut in die Aorta zurückzuführen, während das Herz während einer Herzoperation mit einem Bypass versehen wird. Diese Kanülen haben einen kleinen Durchmesser, typischerweise zwischen 6 mm bis 8 mm, jedoch für pädiatrische Anwendungen noch kleiner, um die Gefahr der Beschädigung der Aorta zu minimieren. Solche Beschädigungen haben bei vielen Herzpatienten zu komplexen ateriosklerosen Verletzungen und damit verbundenen Thrombosen geführt. Die Strömungsgeschwindigkeit durch diese im Durchmesser kleinen Kanülen ist sehr hoch, weil die erforderliche Blutströmungsrate ungefähr 5-7 l/min. beträgt. Dies führt bei rohrartigen Kanülenauslässen dazu, daß der Blutstrom in Form eines scharfen Strahles aus dem distalen Ende der Kanüle in die Aorta strömt. Hierbei besteht die Gefahr, daß der scharfe Blutstrahl Teilchen, insbesondere Ablagerungen (Plaque), von der Aorteninnenwand abreißt und diese weggespülten Teilchen dann eine Embolie verursachen. Zur Vermeidung dieser Nachteile schlägt die bekannte Patentschrift vor, am distalen Ende der Kanüle keine rohrförmige Auslaßöffnung, sondern eine Vielzahl von kleineren Auslaßöffnungen für das Blut zu bilden. Verschiedene Ausführungsformen hierfür sind auch aus den US 5 354 288, 5 616 137 und 5 643 226 bekannt.

Die bekannten Kanülen teilen zwar den Blutstrom in mehrere Einzeiströme auf, jedoch haben diese Einzelströme immer noch eine "Strahl-Wirkung", durch welche Plaque von der Aorteninnenwand abgelöst werden kann.

Durch die Erfindung soll die Aufgabe gelöst werden, eine Kanüle zur Zufuhr von Blut in eine Blutbahn, insbesondere in die Aorta, eines Patienten derart auszubilden, daß der aus dem distalen Ende der Kanüle austretende Blutstrom keine Partikel, insbesondere sklerotischer Plaque, von der Blutbahnwand ablöst, auch dann nicht, wenn das Blut mit sehr hoher Strömungsrate (Volumen pro Zeiteinheit) von der Kanüle in die Blutbahn zugeführt wird.

Diese Aufgabe wird gemäß der Erfindung durch die kennzeichnenden Merkmale von Anspruch 1 gelöst.

Gemäß der Erfindung ist vorgesehen, daß der Strömungsquerschnitt der einzelnen Blutauslaßöffnungen je maximal 0,09 mm$^2$ groß ist, und daß die Summe der Strömungsquerschnitte von allen Blutauslaßöffnungen zusammen mindestens so groß ist wie der Strömungsquerschnitt des mindestens einen Lumens am distalen Ende.

Durch die Erfindung tritt das Blut aus dem distalen Ende der Kanüle in Form eines weichen oder sanften Blutstromes aus, welcher die gleich große Strömungsrate (Volumen pro Zeiteinheit) wie beim Stand der Technik hat, jedoch eine wesentlich niedrigere Strömungsgeschwindigkeit.

Gemäß der Erfindung sind mindestens 30 Blutauslaßöffnungen vorgesehen.

Ein wesentliches Merkmal der Erfindung besteht in der Kombination einer großen Vielzahl von sehr kleinen Blutauslaßöffnungen und der Erzeugung eines Staudruckes in der Kanüle durch die Stege, welche die Wände der Blutauslaßöffnungen bilden. Dadurch wird ein wesentlicher Teil des Strömungsdruckes bereits in der Kanüle auf einen kleineren Wert reduziert, bevor das Blut durch die Blutauslaßöffnungen ausströmt. Beim Ausströmen aus den Blutauslaßöffnungen in die Blutbahn oder Aorta erfolgt eine weitere Reduzierung des Strömungsdruckes durch den größeren Strömungsquerschnitt in dieser Blutbahn bzw. der Aorta. Vorzugsweise ist die erste Reduzierung größer als die zweite Reduzierung des Strömungsdruckes.

Gemäß der Erfindung haben die Blutauslaßöffnungen einen maximalen Strömungsquerschnitt im Bereich zwischen 0,09 mm$^2$ und 0,002 mm$^2$. Vorzugsweise haben alle Blutauslaßöffnungen einen gleich großen Strömungsquerschnitt. Jedoch sind auch Ausführungsformen möglich, bei welchen die Blutauslaßöffnungen verschieden große Strömungsquerschnitte haben.

Die Summe der Strömungsquerschnitte von allen Blutauslaßöffnungen zusammen ist vorzugsweise kleiner als der Strömungsquerschnitt der Blutbahn bzw. der Aorta an der Stelle, wo die Kanüle in sie eingesetzt wird. Die Größe der Strömungsquerschnitte von allen Blutauslaßöffnungen zusammen liegt vorzugsweise in einem Bereich von maximal 13 mm$^2$ für Kleinkinder und 1200 mm$^2$ für Erwachsene. Hierbei wird davon ausgegangen, daß der Durchmesser einer Aorta für Kleinkinder etwa 4 mm und für Erwachsene etwa 40 mm beträgt.

Gemäß bevorzugten Ausführungsformen der Erfindung ist ein Netz vorgesehen, dessen Maschen die Blutauslaßöffnungen bilden. Das Netz ist vorzugsweise ein Gewebe aus sich kreuzenden Fäden, die einen Durchmesser von 30 μm bis 70 μm haben. Dadurch ergibt sich eine sehr dünne Netzdicke von nur ungefähr 30 μm bis 70 μm, und nur an den Kreuzungsstellen der Fäden ergibt sich eine doppelte Netzdicke. Dadurch hat das Netz nur sehr kleine Berührungsflächen für das Blut. Dies ist von großem Vorteil, weil das Blut um so stärker beschädigt wird, je größer und rauher die von dem Blut kontaktierten Flächen sind.

Es werden deshalb Netz-Fäden bevorzugt, deren Durchmesser möglichst klein ist. Selbstverständlich können Netze nicht nur durch Gewebe-Fäden gebildet werden, sondern auch durch gelochte, membranartig dünne Folien.

Das Netz kann eine vom Blutdruck in der Kanüle nicht verformbare Steifigkeit haben. Die bevorzugte Ausführungsform besteht jedoch in einem Netz, welches eine von dem Blutdruck in der Kanüle verformbare Flexibilität hat. Das Netz hat vorzugsweise die Form eines Sackes mit einem Sackboden und einer Sacköffnung, welche letztere mit dem distalen Ende der Kanüle verbunden ist. "Verbunden" bedeutet in dieser Beschreibung die Möglichkeit von zwei Ausführungsformen: eine, bei welcher das Netz und die Kanüle zwei Teile sind und aneinander befestigt sind, und eine andere, bei welcher die Kanüle und das Netz zusammen ein einstückiges Teil sind. Die zweistückige Ausführung kann dabei derart gestaltet sein, daß ein Einführen des Netzes in das distale Ende der Kanüle möglich ist, wenn sich die Kanüle bereits in der Blutbahn des Patienten befindet. Ebenso kann das Netz aus der Blutbahn des Patienten in die Kanüle zuückgezogen werden, während sich die Kanüle noch im Patienten befindet.

Ein flexibles Netz hat den Vorteil, daß es in die Kanüle zurückgeschoben werden kann, damit es beim Einsetzen der Kanüle in eine Blutbahn, z.B. die Aorta eines Patienten, nicht stört und die Blutbahn nicht beschädigen kann. Nach dem Einsetzen der Kanüle in die Blutbahn wird das flexible Netz von dem Druck des Blutes aus der Kanüle hinaus gedrückt.

Das Netz hat vorzugsweise eine von der Sacköffnung zum Sackboden hin kegelförmig enger werdende Form oder mindestens eine halbkugelförmige Form, damit eine sehr große Vielzahl von Blutauslaßöffnungen, z.B. mindestens 30, vorgesehen werden können, über welche Blut in Richtung nach vorne aus der Kanüle ausströmen kann.

Weitere Merkmale der Erfindung sind in den Unteransprüchen enthalten.

Die Erfindung wird im folgenden mit Bezug auf die Zeichnungen anhand von bevorzugten Austührungsformen als Beispiele beschrieben. In den Zeichnungen zeigen

Fig .1    eine Kanüle nach der Erfindung im Axialschnitt, eingesetzt in eine Aorta, mit einem Netz, welches aus dem distalen Ende der Kanüle konvex hinaus gewölbt ist,

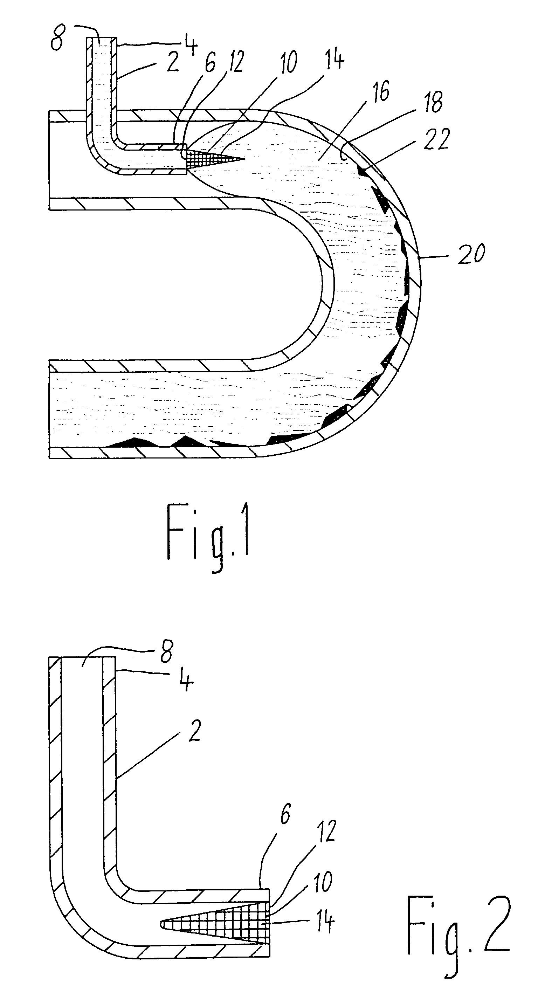

Fig .2    einen Axialschnitt durch die Kanüle von Fig. 1, wobei das Netz in das distale Ende der Kanüle hineingezogen oder hinein gedrückt ist, bevor die Kanüle in die Aorta eingesetzt wurde,

Fig. 3    einen Längsschnitt längs der Ebene III-III von Fig. 4 durch eine weitere Ausführungsform einer Kanüle nach der Erfindung,

Fig. 4    eine Vorderansicht der Kanüle von Fig. 3,

Fig. 5    einen Längsschnitt längs der Ebene V-V von Fig. 6 einer nochmals anderen Ausführungsform der Kanüle nach der Erfindung, und

Fig. 6    eine Vorderansicht der Kanüle von Fig. 5.

Gemäß der Erfindung liegt die Porosität am distalen Ende der Kanüle durch die dort vorgesehenen Blutauslaßöffnungen bei mindestens 25%, vorzugsweise jedoch mindestens 60%. Der bevorzugte Bereich liegt bei ungefähr 40% bis 70%.

Die Porosität ist hierbei nach der folgenden Formel definiert:

$$\text{Porosität} = \frac{d}{f}$$

Hierin bedeuten:

d:    der Strömungsdurchlaßquerschnitt von allen Auslaßöffnungen zusammen;

f:    Gesamtfläche, bestehend aus d und der von den Stegen verschlossenen Fläche

Die in den Fig. 1 und 2 dargestellte Kanüle 2 hat ein proximales Ende 4 und ein distales Ende 6, welche durch mindestens ein Lumen 8 (Kanal) miteinander verbunden sind. Das Lumen hat vorzugsweise einen kreisrunden Querschnitt. Mit dem distalen Ende 6 ist ein Netz 10 verbunden, welches weitgehend den gesamten Querschnitt der Öffnung 12 des Lumens 8 am distalen Ende 6 überbrückt. Die Maschenöffnungen des Netzes 10 bilden Blutauslaßöffnungen 14 am distalen Ende 6 des Lumens 8.

Das Netz 10 kann am distalen Ende 6 durch Befestigungsmittel befestigt sein oder zusammen mit der Kanüle 2 aus einem einstückigen Teil bestehen.

Das Netz 10 hat vorzugsweise eine hohlkegelförmige Sackform, dessen im Durchmesser größerer Kegelrand bzw. die Sacköffnung an der Wand des distalen Endes 6 der Kanüle 4 in Blutströmungsrichtung fluchtend angrenzt.

Die Maschenöffnungen 14 zerteilen einen vom proximalen Ende 4 zum distalen Ende 6 strömenden Blutstrom beim Ausströmen aus der Lumenöffnung 12 in eine sehr große Vielzahl, z.B. mehr als 30, von so eng nebeneinander liegenden Einzelstrahlen, daß diese Einzeistrahlen unmittelbar nach dem Netz 10 zu einem weichen oder sanften Gesamtblutstrom 16 zusammenfließen, bevor das Blut die innere Kanalwand 18 der Aorta 20 trifft. Dadurch wird vermieden, daß in der Aorta 20 gegebenenfalls vorhandener sklerotischer Plaque 22 von dem Blutstrom 16 abgelöst und weggespült wird.

Wie Fig. 2 zeigt, kann das Netz 10 eine vom Blutdruck verformbare Flexibilität haben und vor dem Einführen der Kanüle in die Aorta in das distale Ende 6 des Lumens 8 zurückgeschoben oder zurückgezogen werden, damit es beim Einführen der Kanüle in die Aorta 20 nicht stört und die Aorta nicht beschädigen kann. Wenn nach dem Einführen der Kanüle in die Aorta eines Patienten ein Blutstrom durch die Kanüle in die Aorta zugeführt wird, drückt das Blut das Netz 10 von der in Fig. 2 gezeigten, zurückgesetzten Position in die in Fig. 1 gezeigt, nach vorne überstehende Position, in welcher das Netz 10 aus der Öffnung 12 des Lumens 8 nach vorne hinaus konvex gewölbt ist.

Das Netz 10 besteht vorzugsweise aus dünnen Fäden oder Filamenten. Diese haben vorzugsweise eine Dicke von maximal 80 μm, vorzugsweise jedoch nicht größer als 20 μm. Die Fäden oder Filamente des Netzes 10 kreuzen sich, so daß das Netz an diesen Kreuzungsstellen eine doppelte Dicke hat. Gemäß einer anderen Ausführungsform kann das Netz 10 eine dünne, gelochte Folie sein, an der Kanüle 4 befestigt sein oder zusammen mit der Kanüle ein einstückiges Teil sein.

Die Porosität des Netzes 10 durch die Maschenöffnungen 14 liegt vorzugsweise im Bereich zwischen 25% und 80%, gemäß besonderer Ausführungsform im Bereich zwischen 40% und 70%.

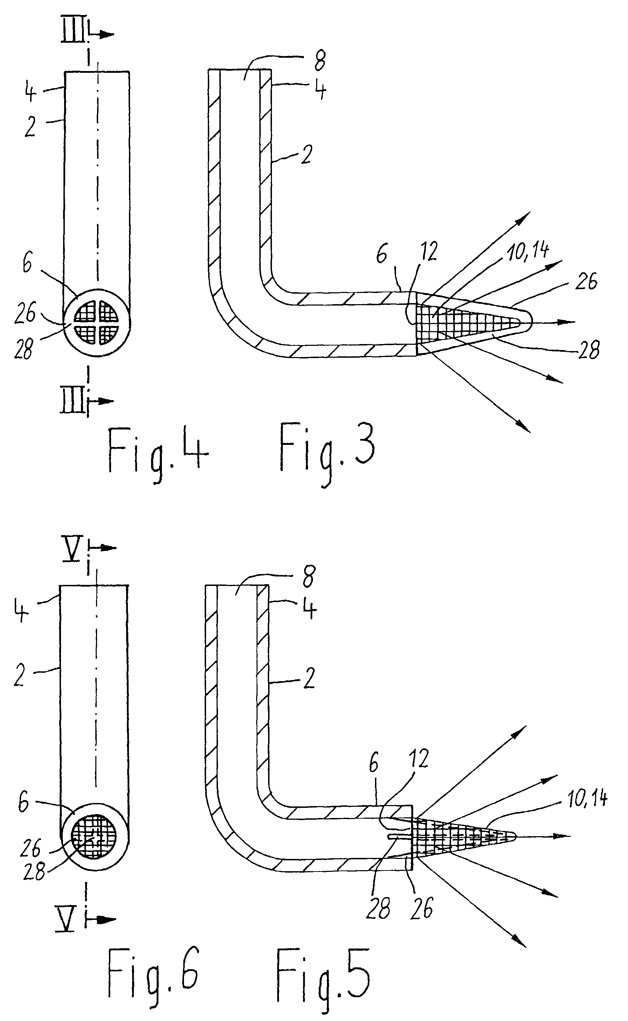

Bei den Ausführungsformen nach den Fig. 3 und 4 ist das Netz 10 in einem seiner Form angepaßten Korbteil 26 angeordnet, welcher die Form des Netzes stabilisiert und das Einführen des distalen Endes 6 mit dem Netz 10 in die Aorta 20 erleichtert sowie Beschädigungen der Aorta durch das Netz 10 verhindert. Der Korbteil 26 besteht aus mindestens 3, vorzugsweise 4 Stegen 28, welche an dem distalen Ende 6 der Kanüle 4 befestigt sind oder mit dieser aus einem einzigen Stück bestehen.

Bei der Ausführungsform nach den Fig. 5 und 6 hat der Korbteil 26 die gleiche Funktion wie in den Fig. 3 und 4, wobei sich das Netz 10 nicht in dem Korbteil 26 befindet, sondern den Korbteil 26 umgibt.

Die Kanüle und der Teil, welcher die Blutauslaßöffnungen bildet, besteht aus einem Material, welches eine geringe Haftreibung an seiner Oberfläche hat, beispielsweise aus Polyethylen, Polyamid oder Polypropylen.

**Patentansprüche**

1. Kanüle mit einem proximalen Ende (4), einem distalen Ende (6), mit einer Vielzahl von Blutauslaßöffnungen (14) am distalen Ende (6), und mindestens einem Lumen (8) zwischen diesen Enden zur Zufuhr von Blut in eine Blutbahn, insbesondere Aorta, eines Patienten, in welche die Kanüle einsetzbar ist, **dadurch gekennzeichnet,** daß der Strömungsquerschnitt der einzelnen Blutauslaßöffnungen (14) je maximale 0,09mm$^2$ groß ist; daß die Summe der Strömungsquerschnitte von allen Blutauslaßöffnungen (14) zusammen mindestens so groß ist wie der Strömungsquerschnitt des mindestens einen Lumens (8) am distalen Ende (6).

2. Kanüle nach Anspruch 1, **dadurch gekennzeichnet,** daß die Summe der Strömungsquerschnitte von allen Blutauslaßöffnungen (14) zusammen größer ist als der Strömungsquerschnitt des mindestens einen Lumens (8) am distalen Ende (6).

3. Kanüle nach Anspruch 1 oder 2, **dadurch gekennzeichnet**, daß der Strömungsquerschnitt der einzelnen Blutauslaßöffnungen (14) je maximal 0,032mm$^2$ groß ist.

4. Kanüle nach Anspruch 1 oder 2, **dadurch gekennzeichnet**, daß der Strömungsquerschnitt der einzelnen Blutauslaßöffnungen je maximal 0,018mm$^2$ groß ist.

5. Kanüle nach Anspruch 1 oder 2, **dadurch gekennzeichnet**, daß der Strömungsquerschnitt der einzelnen Blutauslaßöffnungen je maximal 0,003mm$^2$ groß ist.

6. Kanüle nach Anspruch 1 oder 2, **dadurch gekennzeichnet**, daß die Strömungsquerschnitte der Blutauslaßöffnungen (14) unterschiedlich groß sind und ihre Größe im Bereich zwischen 0,032mm$^2$ und 0,003mm$^2$ liegt.

7. Kanüle nach einem der vorhergehenden Ansprüche, **dadurch gekennzeichnet**, daß die Summe der Strömungsquerschnitte von allen Blutauslaßöffnungen (14) zusammen kleiner als der Strömungsquerschnitt der Blutbahn (20) an der Stelle ist, wo das distale Ende (6) in die Blutbahn einsetzbar ist.

8. Kanüle nach Anspruch 7, **dadurch gekennzeichnet**, daß die Summe der Strömungsquerschnitte von allen Blutauslaßöfffnungen (14) zusammen maximal 700mm$^2$ groß ist.

9. Kanüle nach Anspruch 7, **dadurch gekennzeichnet**, daß die Summe der Strömungsquerschnitte von allen Blutauslaßöffnungen (14) zusammen maximal 314mm$^2$ groß ist.

10. Kanüle nach Anspruch 7, **dadurch gekennzeichnet**, daß die Summe der Strömungsquerschnitte von allen Blutauslaßöffnungen (14) maximal 79mm$^2$ groß ist.

11. Kanüle nach Anspruch 7,

**dadurch gekennzeichnet**,
daß die Summe der Strömungsquerschnitte von allen Blutauslaßöffnungen zusammen maximal 7mm$^2$ groß ist.

**12.** Kanüle nach einem der vorhergehenden Ansprüche,
**dadurch gekennzeichnet**,
daß ein Netz (10) vorgesehen ist, dessen Maschen die Blutauslaßöffnungen (14) bilden.

**13.** Kanüle nach Anspruch 12,
**dadurch gekennzeichnet**,
daß das Netz (10) eine von dem Blutdruck im Lumen (8) nicht verformbare Steifigkeit hat.

**14.** Kanüle nach Anspruch 12 oder 13,
**dadurch gekennzeichnet**,
daß die Ausdehnung des Netzes (10) wesentlich größer ist als der Strömungsquerschnitt des mindestens einen Lumens (8) am distalen Ende (6) und daß das Netz sich sackartig stromabwärts über das distale Ende hinaus erstreckt, wobei die Sacköffnung mit dem distalen Ende (12) verbunden ist.

**15.** Kanüle nach Anspruch 12,
**dadurch gekennzeichnet**,
daß das Netz (10) eine von dem Blutdruck in dem mindestens einen Lumen (8) leicht verformbare Flexibilität hat, daß die Ausdehnung des Netzes (10) wesentlich größer ist als der Strömungsquerschnitt des mindestens einen Lumens (8) am distalen Ende (6), und daß das Netz (10) eine sackartige Form hat, dessen Sacköffnungsrand mit dem distalen Ende (12) verbunden ist.

**16.** Kanüle nach Anspruch 15,
**dadurch gekennzeichnet**,
daß das Netz (10) in dem mindestens einen Lumen (8) untergebracht ist und durch den Blutdruck aus dem ditalen Ende (6) hinausstoßbar ist, wobei der Sacköffnungsrand mit dem ditalen Ende verbunden bleibt.

**17.** Kanüle nach einem der Ansprüche 14 bis 16,
**dadurch gekennzeichnet**,
daß die Sackform des Netzes (10) von dem Sacköffnungsrand zum Sackboden kegelförmig enger werdend ausgebildet ist.

**18.** Kanüle nach einem der Ansprüche 15 bis 17,
**dadurch gekennzeichnet**,
daß ein Stabilisiermittel (26, 28) vorgesehen ist, welches das flexible Netz (10) in seiner sackartigen Form stabilisiert.

**19.** Kanüle nach einem der vorhergehenden Ansprüche,

**dadurch gekennzeichnet**,
daß die zwischen den Blutauslaßöffnungen (14) gelegenen Stege, welche die Öffnungsränder bilden, eine Dicke von maximal 0,2mm haben.

**20.** Kanüle nach Anspruch 19,
**dadurch gekennzeichnet**,
daß die Stege zwischen den Blutauslaßöffnungen, welche die Ränder der Blutauslaßöffnungen bilden, eine Dicke von maximal 0,07mm haben.

**21.** Kanüle nach Anspruch 19,
**dadurch gekennzeichnet**,
daß die Stege zwischen den Blutauslaßöffnungen, welche die Ränder der Blutauslaßöffnungen bilden, eine Dicke von maximal 0,03 mm haben.

Fig.1

Fig.2

Fig.4　　Fig.3

Fig.6　　Fig.5